# EUROPEAN PATENT APPLICATION

(11) **EP 1 757 303 A1**
(43) Date of publication of application: **28.02.2007**
(21) Application number: 05752318.5
(22) Date of filing: 09.06.2005
(51) Int. Cl.: A61K 38/43, A61K 9/12, A61P 31/04, A61P 31/12

(54) **HUMAN LYSOZYME MEDICINE, ITS MANUFACTURING METHOD AND APPLICATION THEREOF**

(30) Priority: 10.06.2004 CN 200410020738
(71) Applicant: An, Mi, Liaoning 116600 (CN)
(72) Inventor: An, Mi, Liaoning 116600 (CN)
(74) Representative: Jannig, Peter
(86) International application number: PCT/CN2005/000822
(87) International publication number: WO 2005/120556

(57) **Abstract**

The present invention relates to biomedicine, in particular, relates to human lysozyme medicine in the form of aerosol, which contains 1,500-3,000,000U/ml human lysozyme, and its manufacturing method. In a certain embodiment of the invention, the medicine consist of recombinant human lysozyme which activity is at the range of 15,000u-300,000U/ml, 0.28 span85, 0.28g oleic and acetic acid, 10g 134A, 80% 10-20mM (pH6.5-07.5) phosphate buffer, and 5-25% propylene glycol. The medicine of the invention can be used to preventing and treating the pneumonia, tracheitis, faucitis or amygdalitis, which is caused by virus, bacterium, drug-resistant bacteria or chlorine, and can be used to treating tracheitis, pneumonia, or abscess of lung, which is caused by virus or SAES. Compared to other form, the present human lysozyme medicine in the form of aerosol is more convenient in administration, and can enhance curative effect, and has no toxic side effect.

## Description

### Technological field

The present invention relates to biomedicine, in particular, relates to human lysozyme medicine in the new form, pharmacy field and its application

### Background technology

The antibiotic has created a lot of medical miracles, make a lot of diseases disappear, for instance pneumonia, meningitis, puerperal fever, septicaemia, tuberculosis, etc.. Today in the 21st century, the development drug-resistance bacteria make people shocking. So-called drug-resistance bacteria is the resisting of the medicine produced by bacterium. After the bacterium keeps in touch with the medicine many times ,bacterium showed the low susceptibility to the medicine. The drug-resistance bacteria includes staphylococcus aureus (MRSA) , staphylococcus epidermidis(MRSE), Streptococcus pneumoniae, hemolytic streptococcus , enterococci, Escherichiacoli, Klebsislla pneumoniae, propionibacterium , statobacillus, ci-tricbacillus, Serratia , Aerobacter cloacae, pseudomonas aeruginosa, albedocandida, etc. The drug-resistance mainly exists in the following medicine :clarithromycin, roxithromycin, amoxicillin, vancomycin, Declomycin, penbritin, cefuroxime, benzylpenicillin sodium, benzylpenicillin potassium, sulfonamides.

Concrete standardization of drug-resistance (diameter of bacteriostasis circle nm)

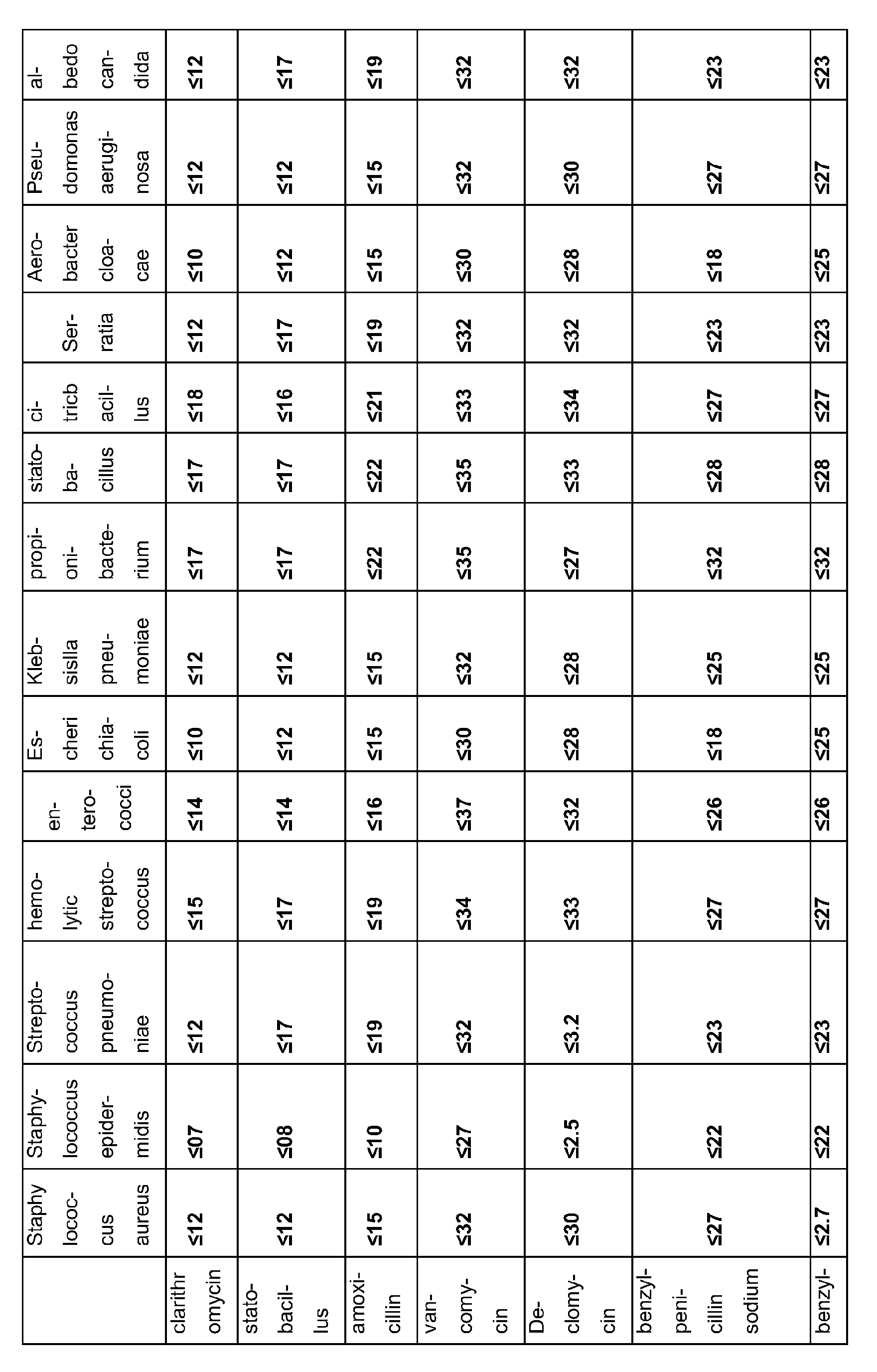

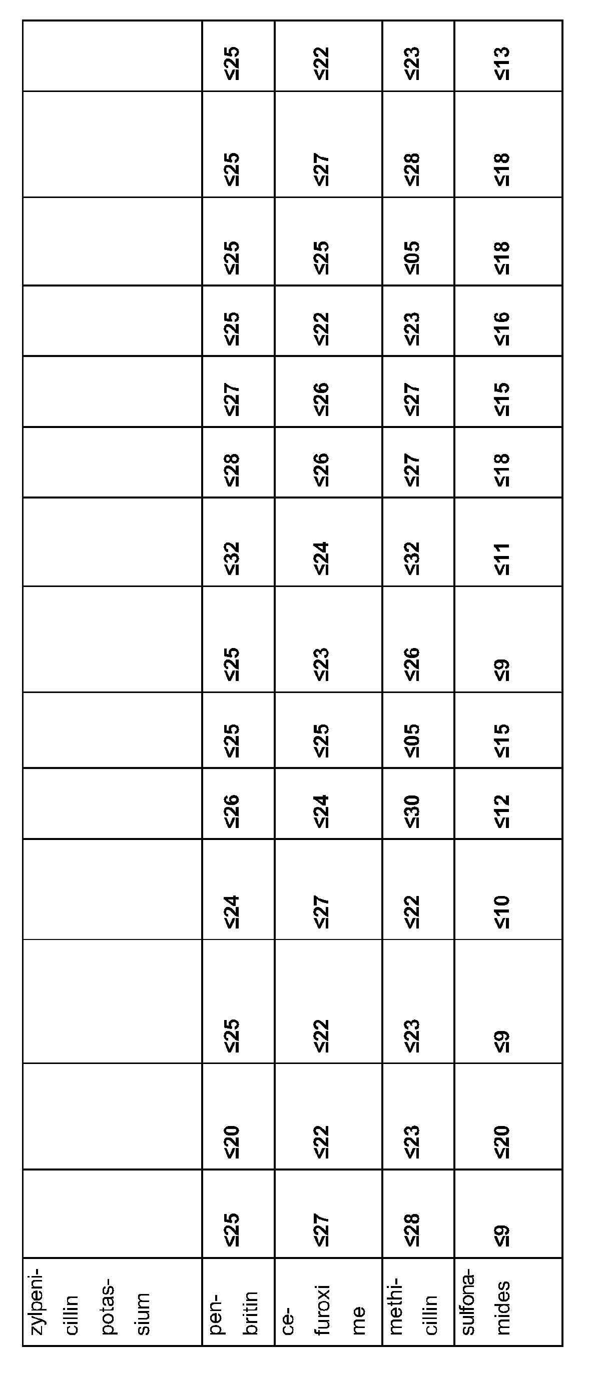

From the history of bacteria resistance, after a new generation of antibiotic appears, a batch of new drug-resistance bacteria will appear. It takes 10 years or so to develop a kind of new antibiotic, but the production of a new generation of drug-resistance bacteria needs only 2 years. The research speed of the antibiotic can not far catch up with the development speed of the drug-resistance bacteria. At present, a new "super antibiotic" effective to different drug-resistance bacteria was badly needed to be developed for clinical treatment. Recombinant human lysozyme is a well known bacteriolytic enzyme which name is 1,4-β-N-lysozyme or peptidoglycan N- acetylmuramylhydrolase .It hydrolyzes β-1,4 glycoside bonds between N-acetylmuramic acid and N-acetylglucosamine in peptidoglycan of the bacterial cell wall. Because of its bactericidal activity, lysozyme has been of interest as an anti-virus, anti- tumor anti-inflammation and immunological regulation agent in medicine. China patent 03110824.5 recorded recombinant human lysozyme medicine has an obvious curative effect of drug resistance enzyme and anti-virus. But its form of a medicine is ordinary form. The form is a very important factor to influence the absorption and indication. How to choose and realize the best dosage form can further improve the use of medicine.

### Invention content

The purpose of this invention is overcoming the above-mentioned deficient questions, offering a kind of the human lysozyme medicine. The form of medication is rational, remarkable, easy to absorb, convenient to use. In addition simple manufacturing method is offered. Finally offer its application in medicine, and diseases prevention.

The technological scheme adopted in order to realize above-mentioned purposes in this invention is human lysozyme medicine and the form of aerosol.

The medicine contains 1500-3000000 U/ml human lysozyme.

The medicine consists of recombinant human lysozyme which activity is at rage of 15000u-300000U/ml, 0.28g span85, 0.28g oleic and acetic acid ,10g 134A,80% 10-20 mM(PH 6.5-7.5) phosphate buffer, and 5-25% propylene glycol.

The human lysozyme is recombinant human lysozyme which is expressed by genetic engineering. Genetic engineering expresses human lysozyme's amino end (aminoglutaminic acid - 2-aminopropionic acid)₂, or (aminoglutaminic acid - 2-aminopropionic acid)₃, or genetic engineering express or chemical synthesis mutant recombinant human lysozyme.

Preparation technology stated as follows:
Make 95%-99% purity and 30000 U/mg activity freeze-dried recombinate human lysozyme powder into 15000-3000000 U/granule aerosol capsule, the capsule inspires the spray pump, according with pharmacy rules in the pharmacy factory of GMP.
Make 95%-99% purity and 30000 U/ml/mg activity recombinate human lysozyme into 15000-3000000 U/mL/piece aerosol capsule, according with pharmacy rules in the pharmacy factory of GMP.
Aerosol should be disposed under avoiding the bacterium environment, various kinds of apparatus, container, etc. must be clean, sterilization. Pay attention to prevent pollution of microorganism in the whole procedure, according with pharmacy rules in the pharmacy factory of GMP.
Preparation technology stated as follows: handle and assemble of container and valve-preparation and assignment of medicine - impact propellants-quality control - product (according with pharmacy rules in the pharmacy factory of GMP)
First, treatment of the container: Accord with pharmacy rules in the pharmacy factory of GMP request, finish cleaning, sterilization, drying the aerosol can. Adopt the aerosol pump, use the stifling sterilization of chloroform.
Second , preparation and assignment of medicine: More than 95% purity of recombinant human lysozyme, active 15000u-3000000 U/ml recombinant human lysozyme solution, 0.28g span85 , 0.28g oleic and acetic acid 10g 134A,80% 10-20 mM(PH 6.5-7.5) phosphate buffer, and 5-25% propylene glycol, misce bene under normal atmospheric temperature, compound into 15000u- 3000000U/ ml product (according with pharmacy rules in the pharmacy factory of GMP) .After disposing according to the disperse system, undergoing quality examination separately, quantitative partial assignment, install valve, seal the cap.
Third, impact propellants: The method of impacting propellants is pressing can , load container of shipment medicine onto the valve, seal the cap, release the air in the container first , so as not to influence the pressure of the container, then irritate propellants into the can through the pressure. Assay the product.
This human lysozyme medicine of the invention can be used to preventing and treating diseases caused by the virus, the bacterium, drug-resistant bacteria or chlorine.

This stated human lysozyme medicine can apply in preventing and treating the peneumonia, tracheitis, faucitis or amygdalitis, which is cause by virus, bacterium, drug-resistant bacteria or chlorine.

This stated human lysozyme medicine can be used to treating tracheitis, faucitis, or abscess of lung, which is caused by virus or SAES.

The new form of recombinant human lysozyme have developed in this invention, compared with other form medicine have more outstanding characteristics: 1, the lung has the greater surface area of absorption and thinner absorption layer. Alveoli pulmonis surface area receive for 100m2, and alveoli pulmonis upper skin very thin, cover with capillary, this not only can make the little member gas exchange fast but also can absorb a lot of macromolecule medicines . 2, the activation of metabolic enzyme in the lung is relatively low, there are no existence and interference of different digestive enzyme, enable the polypeptide and protein medicine to exist steadily, after the medicine is absorbed it does not pass the liver, which can reduces the first-pass effect. 3, the lung environment is steady, the time of medicine staying is relatively long there can be good bioavailability to slow-absorb medicine. 4, the use of aerosol is free of environment influence, the technology of the equipment is simple, it is convenient and shortcut. The lung is usually thought to fast clear up the medicine, contribute rapidly. The diameter of atomization gas of compressing the atomization inspiring machine is regular in 1-5 microns , 70% gas between atomized 1-3 microns of diameter of particle, so 70% of the medicines can reach lower respiratory tract and lung , can reach the ideal clinical therapeutic efficacy. The rational the form of medicine has widenned the application of recombinant human lysozyme, strengthened the therapeutic efficacy, safe and no side effect; Its simple preparation technology gives medicines in the form of aerosol, which is more convenient in administration, and can enhance therapeutic efficacy.

In order to better understand the essence of this invention, We will illustrate the new use of gene recombinant Human Lysozyme in the pharmaceutical field through the pharmacological test and its result.

Gene recombinant Human Lysozyme was prepared with 200mL medium, phosphonic acid 4-8mL, magnesium sulfate 1-5g, potassium sulfate 2-6g, potassium hydroxide1-3g, calcium sulfate1-3.5g were added, then added water to 200mL. After autoclaving strain was inoculated, the inoculate rotation speed of shaker was 250 r/min, culture temperature was 20-35 °C, cultured in the constant temperature bed for 36-48 hour, cultured in the seeding tank then cultured in the fermenter. The culture solution which has completed fermentation was extracted and purified, then lyophilize the concentrated solution. Examined the protein, purity, tested the activity of lysozyme . Make valid gene recombinant Human Lysozyme into the powder or the solution into 1500U- 30000U/ ml aerosol.

First. Model test to mice:
A, atomization sucks gene recombinant Human Lysozyme (HLZ) cured bronchus pneumonia mice infected by Staphylococcus aureus MRSABAA-42
Medicine and reference medicines
Medicine: the gene recombinant Human Lysozyme (Human Lysozyme, HLZ): active unit 30000u/mg, batch number 020110, provided by the Biochemistry Institue of Dalian Qilong, prepare into the necessary concentration with PBS before the experiment.
Reference medicines: Clarithromycin: potency 948U/ mg, National Institute for the Control of Pharmaceutical and Biological Products.
Experiment strain: Staphylococcus aureus MRSABAA-42 isolated from clinical laboratory in 2002 was present by the China's Western medicine center of university of Sichuan.
Animals: Kunming mice, mean body weight 17-20g, were purchased from animal center of Sichuan antibiotic industrial research institute, animal's certificate: Sichuan experimental anima quality management No. 99-30 (2003)

### Instrument:

<1> Atomization machine core, type 46mm, it is producd by Ningbo qinzhou tangxi hongda electric apparatus fittings factory, fix it in stainless steel cup (Φ12cm), using for making the mould of atomization inspiring hydrochloric acid.
<2> Atomizer for treatment: The compression sprayer (PARL.BOY) produced in Berry company, Germany for the treatment of atomization of human lysozyme. <3> Type BL3100, Electronic balance (type BS 200S-WEI) produced in Beijing Sartorius balance company.
<4>Glass desiccator: Φ23×12.5cm.

The treatment experiment of bacterial pneumonia mice
(1) Preparing the bacterium solution and modeling
Choose the test lawn, after washing with the physiological saline, correcting by McB tubes for comparative tests bacterium solution concentration 2#MCF, turbidity of 2.7×10¹¹CFU / ml , diluted with physiological saline separately into 10⁻¹, 10⁻², 10⁻³ bacterium solution , drip the nose to infect for mice respectively (anaesthetize the mice with the pentobarbital first, 30mg/kg, ip.), 0.05 ml a mouse. Observed the mice body changing, and killed parts of mice after infecting 48h, fetch the whole lung to tissue homogenate, 10 times diluted by physiological saline, draw 0. 1m1 to MH agar plate surface, to cultivate last night 35 , Gram staining, test under microscope, and count plate. At the same time recorded the number of death mice, and collect some mice tracheas , bronchus , lung tissue and do pathology tissue observing.
The bacterium culture was positive, and the bacterium counted ≥100CFU/ ml in the lung. It was obviously observed that pathology histology change in mice trachea , bronchus , lung tissue , such as synathroisis , hydroncus, inflammatory cell infiltrate ,etc.. At the same time mice death rate above 50% indicated that modeling succeeded.
It is called medial lethal dose (LD₅₀) that bacterium amount caused mice appearing lung pharmacology change and 50% died. LD₅₀ was used as experimental treatment amount.
(2)Infected mice and grouping:
Choose health Kunming mice, body weight 17-20g, anaesthetize with the pentobarbital (30mg/ kg , ip.), drip the nose to infect for mice respectively(0.05 ml a mouse) drawing 10 times of LD₅₀ bacterium solution ,method is same as above, infected 300 altogether. Infected miceis divided into 10 groups at random according to the weight, 30 every groups.
(3)The drug treatment:
Designed dosage: The concentration of recombinant human lysozyme medicine was 15000u/ml as in clinical atomization therapy. Its dose was 15000u per time and administrated one time everyday, I.e 0.5mg per time everyday. The dose was 1.3×10-3mg a mice (counted by weight of 20g). Human ventilatory capacity (9000ml/min) corresponded with 375 times of mice ventilatory capacity (24ml/min). Human single dose (0.5mg/70kg) corresponded with 384 times of mice dose (1.3×10-3mg/20g).Two doses were closed, so clinical human dose was used for atomization therapy of mice. Availability of clinical inspiring lysozyme atomized was higher than breathing amount of mice by itself in atomizer. To ensure the breathing amount of lysozyme, the breathing time were extended for 0.5 hour.Based on 0.5mg/ml, the concentration of 0.25 mg/ml ,1.0mg/ml and 2.0mg/ml were set respectively as treatment dose. Result is shown in Tab. 1-1.

**Tab. 1-1 atomization recombinant human lysozyme experiment grouping**

| No. | Dosage | Numbers of animal |
|---|---|---|
| 1.Normal group | Equal volume NS | 30 |
| 2.lnfected control group | Equal volume NS | 30 |
| 3.Clarithromycin group | 10 | 30 |
| 4.Clarithromycin group | 5 | 30 |
| 5.Clarithromycin group | 2.5 | 30 |
| 6. Clarithromycin group | 1.25 | 30 |
| 7. Human Lysozyme group | 2 | 30 |
| 8. Human Lysozyme group | 1 | 30 |
| 9. Human Lysozyme group | 0.5 | 30 |
| 10. Human Lysozyme group | 0.25 | 30 |

The same group mice were put into the ultrasonic atomizer and then treated with human lysozyme atomized in the compression sprayer (PARL.BOY), twice a day, 5 days in succession.
(4)Detection method:
Every group, from the infected day to calculae, the animal incidence and death were recorded 14 days in succession. Every group collected 2-3 mice and killed them the 14th day after infecting, did bacteriology detection after lung homogenate, killed surplus animal to fetch the trachea , bronchus , fixed in 10% of the formaldehyde solution after weighing, did pathology check.
The bacterial culture: A part of mice were killed after infecting 24h, fetched the whole lung weighting, added 2ml degerming PBS solution 10 times diluted, draw 0. 1 mL to MH agar plate surface, to cultivate last night 35 , counted in the cultivation plateform of 30-300CF, if the two close level tissue fluid is 30 --300CFU, counted the low density (make 3 plateforms at the same time each tissue fluid, calculate the average). Collected 2 mice to do bacterial culture in every group at random in 5,7,14 days after administration.
Pathology detection: After dissecting the mice, observed the trachea, bronchus, syncretio of lung, chest wall and membrana pleuralis, the volume of lung and size of lung focus, the lung surface was congested, hydroncus, consolidation and atelectasis by the naked eye. Took out the trachea , bronchus and the whole lung tissue to fixed with 10% of the formaldehyde solution, draw materials. Dehydrating, the paraffin imbedding, cut into slices, HE dyeing, observes the pathology change of trachea , bronchus , lung under light microscope .
Death rate, and median effective dose calculation (ED₅₀) : since infecting, death and survival rate were recorded 14 days in succession, average survive days, comparing with infect control group. According to Bliss, use NDST software to calculate ED₅₀ value and 95% of the feasible limits.

### Result

The bacterium culture: A part of mice lung tissues grinding solution was carried on bacteriology detection on the 3rd, 7th and 14th day after affected, and the result of bacteriology detection is shown in Tab. 1. Atomization inspired the recombinant human lysozyme (HLZ) 2, 1, 0.5mg/ ml, lung bacterial number was significance lower than infected control group on the 1st, 3rd ,5th day. The bacterial number obviously reduced as increasing day of medicine, having significance differences (P<0. 05).By the 14th day, the recombinant human lysozyme group bacterium basically turned to be negative, ≤10CFU/ ml.

The result of pharmacology histology : The trachea , bronchus , lung tissue of the gene recombinant human lysozyme 2 , 1 , 0. 5mg/ ml group and infected model group were dissected on the 3th,7th day, the result of pathology detection was seen in Tab.2, 3 and 4. The result indicated that the human lysozyme group's trachea , bronchus , lung disease degree was obviously lower than infected model group's. Mice were killed on the 7th, 14th day after administration and observing finish 24h, did pharmacology histology detection of lung tissue. The trachea , bronchus , lung tissue of the human lysozyme group turned to normal basically, and 80% animals of infected model group died, a few surviving mice were close to death, pathology changed such as the focal alveolar wall tissue thickening, part or the disperse distributing monocaryon and the lymphocyte infiltrating , part bleeding , hydroncus ,etc..

Survival rate and ED₅₀ value: The mice infected bronchial pneumonia by staphylococcus aureus (MRSA BAA-42) atomization inspired the recombinant human lysozyme (HLZ) 2, 1, 0.5mg/ ml ,survival rate was 76.7% , 56.7% , 46.7% and 40%, average survive days were 12.93±2.05 day, 11.23± 3.36 day, 3.77±10.3 day, 9.30±4.08 day, significance higher than infected control group in survival rate and days (improving 30-60%, p<0.05). The value of ED50 is 0.53mg/ml (0.20-0.90mg/ml ), seen Tab. 5 and Tab.6.
Result indicated that the mice infected bronchial pneumonia by staphylococcus aureus (MRSA BAA-42) atomization inspired the gene recombinant human lysozyme obtaining good therapeutical effect, the value of ED₅₀ is 0.53mg/ ml . Atomization inspired 2, 1, 0.5, 0.25mg (ml·time) ⁻¹, the survival rate is 76.7%, 56.7%, 46.7% and 40%. Average survival days were 12.93±2.05 days, 11.2± 3.36, 10.3±3.77 days , 9.30±4.08 days, there was significance difference (P<0.05 ) between infected control group. The above-mentioned experimental result indicated atomization inspired the gene recombinant human lysozyme had better therapeutical effect to mice bacterial infection bronchus bronchial pneumonia.

**Tab.1 Result of bacteriology detection of the mice pulmonary infection by taphylococcus aureus MRSA BAA-42 inspired the human lysozyme**

| bacterium | medicine | atomization inspired | animal number | bacterial number(CFU/mL)(n=3) | | | | |
|---|---|---|---|---|---|---|---|---|
| | | | | 0 day | the 1th day | The 3rd day | The 5th day | the 14th day |
| staphylococ cus aureus MRSA BAA-42 5.0×10⁸ | Human lysozyme | 2(60000u/piece) | 5 | 350± | 253.80 | 165.80± | 76.70±11 | 2.50±1.4 |
| | | | | 21.5 | ±22.88 | 16.08^{ΔΔ} | .60^{***ΔΔ} | 6^{***} |
| | | 1 (30000u/piece) | 5 | 350 ± | 254.20 | 172.70 ± | 78.30±1 | 3.5±0.20^{***} |
| | | | | 21.5 | ±25.42^{***} | 17.62 ^{**ΔΔ} | 7.86^{**ΔΔ} | |
| | | 0.5(15000u/piece) | 5 | 350± | 350.60 | 241.90± | 134.60± | 57.60±1 |
| | | | | 21.5 | ±21.5 | 16.62 | 13.58 | 5.37^{**} |
| | Clarithromycin | 6.0 | 5 | 350± | 241.9± | 134.60± | 57.60±1 | 10.0±8.0 |
| | | | | 21.5 | 16.62 | 13.58 | 5.37^{**} | 0^{**} |
| | | 3.0 | 5 | 350± | 246.30 | 141.10± | 60.70±1 | 24.0±2.0 |
| | | | | 21.5 | ±19.03 | 18.54 | 3.98 | 0^{***} |
| | | 1.5 | 5 | 350± | 247.10 | 146.90± | 64.40±1 | 28.0±2.6 |
| | | | | 21.5 | ±21.31 | 13.55 | 5.69 | 0^{**}* |
| | Infected conctrol | ---- | 5 | 350± | 275.60 | 194.30± | 86.70±1 | 130.1±1 |
| | | | | 21.5 | ±21.60 | 17.60 | 2.88 | 93.4 |

| | | | | | | | | |
|---|---|---|---|---|---|---|---|---|
| Compared with infected control group: *P<0.05, **P<0.01 Compared with Clarithromycin group(0.75 mg/ml): ^{Δ}P<0.05, ^{ΔΔ}P<0.01 | | | | | | | | |

**Tab.2 Result of pathology detection of the mice lung tissue**

| Group | dosage of atomization inspired(mg/ml) | time | result of pathology detection |
|---|---|---|---|
| Normal group | equal volume NS | | the alveolar wall tissue thickening, part or the disperse distributing monocaryon and the lymphocyte infiltrating , part bleeding , hydroncus ,etc.. were not seen. |
| Infected model group | equal volume NS | 3rd day | the alveolar wall tissue thickening, monocaryon and the lymphocyte infiltrating |
| | | 7th day 7th day | the disperse alveolar wall tissue thickening, mono-caryon and the lymphocyte infiltrating |
| | | 14th day | the focal alveolar wall tissue thickening, mono-caryon and the lymphocyte infiltrating, part bleeding , hydroncus |
| Human lysozyme group | high dose* (2) | 3rd day | part of alveolar wall tissue thickening, few mono-caryon infiltrating |
| | | 7th day | normal basically |
| | | 14th day | normal basically |
| | medium dose (1) | 3rd day | bronchiole alveolar wall tissue thickening, few monocaryon infiltrating |
| | | 7th day | few monocaryon infiltrating |
| | | 14th day | normal basically |
| | low dose (0.5) | 3rd day | the disperse alveolar wall tissue gently thickening, few monocaryon infiltrating |
| | | 7th day | the part or disperse alveolar wall tissue gently thickening, few monocaryon infiltrating |
| | | 14th day | bronchiole alveolar wall tissue gently thickening, few monocaryon infiltrating |

| | | | |
|---|---|---|---|
| Human lysozyme group: high dose 2mg/ml, medium dose 1 mg/ml, low dose 0.5 mg/ml | | | |

**Tab.3 Result of pathology detection of the mice bronchus**

| Group | dosage of atomization inspired | time | result of pathology detection |
|---|---|---|---|
| Normal group | equal volume NS | ----- | lumens regulation, intact mucous membrane, no bleeding, phlegmasia cell infiltrating |
| Infected model group | equal volume NS | 3rd day | mucous membrane necrosis and shedding, intracavitary full of liquor puris |
| | | 7th day | mucous membrane necrosis and shedding, intracavitary full of liquor puris |
| | | 14th day | mucous membrane necrosis and shedding, intracavitary full of liquor puris |
| Human lysozyme group | high dose (2) | 3rd day | bronchus moderate inflammation, mucous membrane shedding and bleeding |
| | | 7th day | bronchus moderate inflammation, intact mucous membrane, few phlegmasia cell infiltrating |
| | | 14th day | Normal basically |
| | medium dose (1) | 3rd day | bronchus moderate inflammation, bleeding, hydroncus, rhodocyte in intracavitary, lumens phlegmasia cell infiltrating |
| | | 7th day | bronchus moderate inflammation |
| | | 14th day | bronchus moderate inflammation |
| | low dose (0.5) | 3rd day | bronchus moderate inflammation |
| | | 7th day | bronchus moderate inflammation |
| | | 14th day | bronchus moderate inflammation, bleeding |

| | | | |
|---|---|---|---|
| Human lysozyme group: high dose 2mg/ml, medium dose 1 mg/ml, low dose 0.5 mg/ml | | | |

**Tab.4 Result of pathology detection of the mice trachea**

| Group | dosage of atomization inspired | time | result of pathology detection |
|---|---|---|---|
| Normal group | equal volume NS | ---- | lumens regulation, intact mucous membrane, no part bleeding, phlegmasia cell infiltrating |
| Infected model group | equal volume NS | 3rd day | mucous membrane necrosis and shedding, intracavitary full of liquor puris |
| | | 7th day | mucous membrane necrosis and shedding, intracavitary full of liquor puris |
| | | 14th day | mucous membrane necrosis and shedding, intracavitary full of liquor puris |
| Human lysozyme group | high dose* (2) | 3rd day | acute moderate inflammation |
| | | 7th day | Normal basically |
| | | 14th day | Normal basically |
| | medium dose (1) | 3rd day | acute moderate inflammation |
| | | 7th day | acute moderate inflammation |
| | | 14th day | Normal basically |
| | low dose (0.5) | 3rd day | acute hyperphlogosis |
| | | 7th day | acute hyperphlogosis |
| | | 14th day | acute mild inflammation |

| | | | |
|---|---|---|---|
| Human lysozyme group: high dose 2mg/ml, medium dose 1 mg/ml, low dose 0.5 mg/ml | | | |

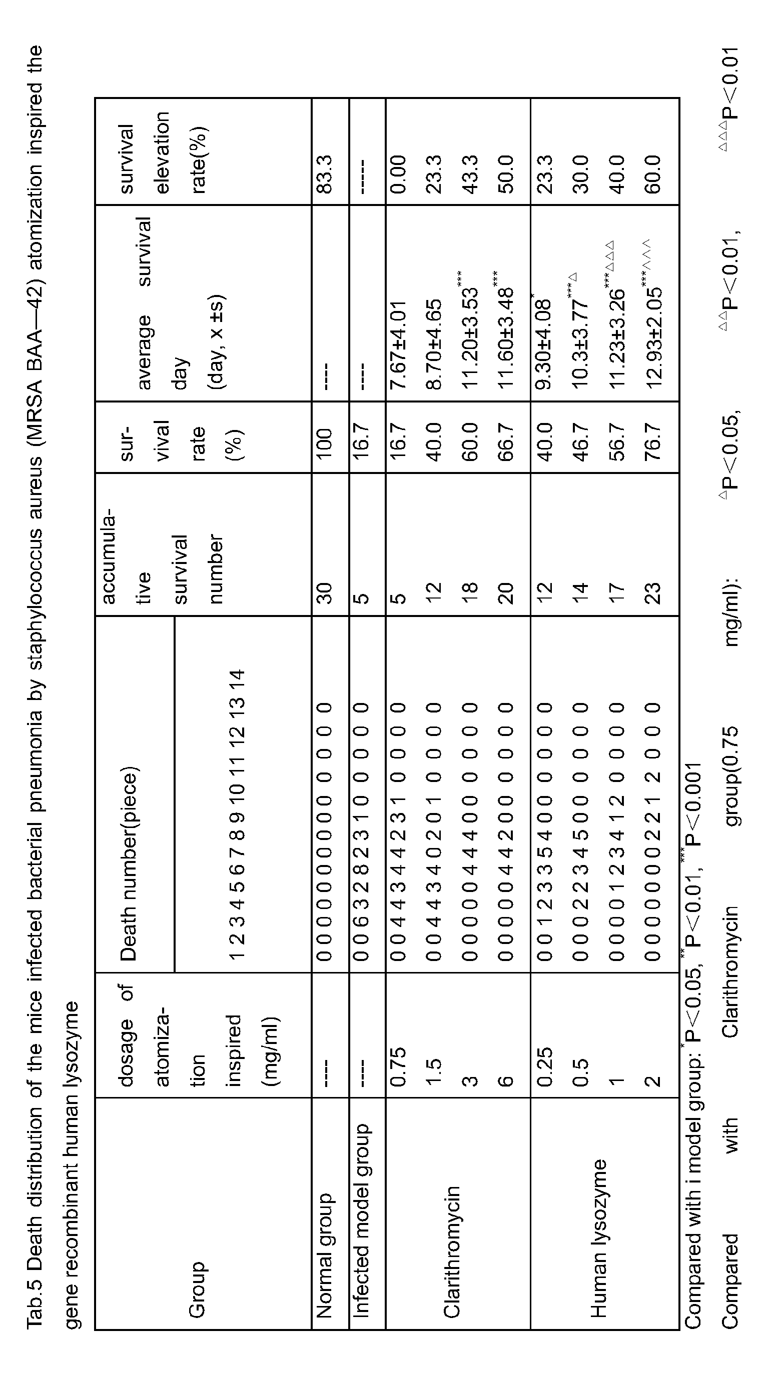

**Tab.6 The therapeutical effect of the mice pulmonary infection by staphylococcus aureus (MRSA BAA-42) atomization inspired the gene recombinant human lysozyme**

| | | | | The 5^{th} day | The 4^{th} day | |
|---|---|---|---|---|---|---|
| Human lysozyme | 2 | 30 | 23 | 76.70±11.60 | 3.5±2.0 | |
| | 1 | 30 | 17 | 78.30±17.86 | 5.5±3.2 | 0.53 |
| | 0.5 | 30 | 14 | 80.80±14.28 | 5.0 ± 3.0 | 0.20-0.90 |
| | 0.25 | 30 | 12 | Nt | Nt | |
| Clarithromycin | 6 | 30 | 20 | 57.60 ± 15.37 | 2.2 ± 0.12 | |
| | 3 | 30 | 18 | 60.70 ± 13.98 | 2.5 ± 0.22 | 2.55 |
| | 1.5 | 30 | 12 | 64.40 ± 15.69 | 3.4 ± 0.25 | 1.76 - 4.02 |
| | 0.75 | 30 | 5 | Nt | Nt | |
| Infection reference | | 30 | 5 | 86.70±12.88 | 64.3±10.82 | |

### B. Preparation of the acute or chronic suppurative faucitis model of little mice infected by the drug-resistant suppurative streptococcus with the upper respiratory tract :

10 healthy Kunming mice (18~22g), male and female, were infected by the drug-resistant suppurative streptococcus with the upper respiratory tract in way of spraying which induced the acute or chronic suppurative faucitis of mice, and then promptly treated with aerosol (powder or liquid) at the dose of 30000u / ml / 20g and give medicines three times a day. Observe for one week after infecting and record the efficient rate of mice. The experimental result indicates that the atomized solution and inhalation of recombinant human lysozyme have obvious curative effect to the acute or chronic suppurative faucitis of mice infected by the drug-resistant suppurative streptococcus with the upper respiratory tract. The results is as follows:

| Disease | Test animal count | Healing | Valid | Invalid | Efficient % |
|---|---|---|---|---|---|
| Acute or chronic suppurative faucitis | 10 | 8 | 2 | 0 | 100 |

### C. The preparation of the amygdalitis model of mice infected by the drug-resistant suppurative streptococcus with the upper respiratory tract :

10 healthy Kunming mice (18~22g), male and female, infected by the drug-resistant suppurative streptococcus with the upper respiratory tract in way of spraying which induce the amygdalitis of mice, then promptly suck aerosol (powder or liquid) according to 30000u / ml / 20g and give medicines three times a day. Observe for one week after infecting and record the efficient rate of mice. The experimental result indicates that the atomized solution and inhalation of recombinant human lysozyme have obvious curative effect to the amygdalitis of mice infected by the drug-resistant suppurative streptococcus with the upper respiratory tract. The results is as follows:

| Disease | Test animal count | Healing | Valid | Invalid | Efficient % |
|---|---|---|---|---|---|
| Amygdalitis | 10 | 8 | 2 | 0 | 100 |

### D. The effect of human lysozyme to bronchus pneumonia of mice by sucking the hydrochloric acid:

### Experimental materials

1, Animal: 50 healthy Kunming mice (about 26g), male, were offered by laboratory animals centre of Sichuan antibiotics industrial institute, quality according with the first class standard, licence number: 005.
2, Test medicines: human lysozyme is the white powder. Batch No.: 020110, potency: 30000u/mg, provided by the Biochemistry Institue of Dalian Qilong, prepared into the required concentration with the physiological saline before using.
3, Reagent: AR grade hydrochloric acid was purchased from market and prepared into I N with the distilled water for use.
4, Instruments:
   <1> Atomization machine core, type 46mm, it is producd by Ningbo qinzhou tangxi hongda electric apparatus fittings factory, fix it in stainless steel cup (Φ12cm), using for making the mould of atomization inspiring hydrochloric acid.
   <2> Atomizer for treatment: The compression sprayer (PARL. BOY) was produced in Berry company, Germany for the treatment of atomization of human lysozyme.
   <3> Type BL31 00 and type BS 200S-WEI Electronic balances were produced in Beijing Sartorius balance company.
   <4> Glass desiccator: Φ23×12.5cm.

### Experimental methods:

50 mice were equally divided to 5 groups at random, selecting randomly 4 groups among them to make the model of the mice atomized hydrochloric acid. Principle of making model:
Each batch of 10 mice was put in Glass desiccator which equipped the self-made atomizer contained 1 N HCl solution. The principle of electronic supra-frequency shaker was used to atomizate hydrochloric acid, then it was inspired for 1 hour by mice to make model of inspiring bronchus lung injury. Its mechanism is that inspiring acidoid can cause adhesion of leucocyte and increasing expression of ICAM-1 and permeability of endo-theliocyte and cellula epithelialis to protein, thereby to cause pneumochysis. Otherwise inspiring acidoid can cause the release of phlegmonosis cytokine. Assembling in lung and reciprocal activation of these cytokine and neutrophilic leukocyte can induce cell adhesion and tissue damage.

40 mice models were gathered and then randomly divided to 4 groups. 3 groups were treated with drug, 1 group were treated as model group.

### Atomization therapeutics:

The concentration of recombinant human lysozyme medicine was set at 15000u/ml in clinical atomization therapy. The dose was 15000u per time once daily, I.e 0.5mg per time. The dose was 1.3×10⁻³mg a mice (calculated by weight of 20g). Human ventilatory capacity (9000ml/min) corresponded with 375 times of mice ventilatory capacity (24ml/min). Human single dose (0.5mg/70kg) corresponded with 384 times of mice dose (1.3×10⁻³mg/20g). Two folds between ventilatory capacity and single dose were closed, so clinical human dose was used for atomization therapy of mice. Availability of clinical inspiring lysozyme atomized was higher than breathing amount of mice by itself in atomizer. To ensure the breathing amount of lysozyme, the breathing time were extended for 1 hour. Based on 0.5mg/ml as low dose, the concentration of 1.0mg/ml and 2.0mg/ml were set respectively as middle and high dose. The mice were put into the glass desiccator and then treated with human lysozyme atomized in the compression sprayer (PARL. BOY) once daily, and continued 5-10 days (shown in Fig. 2).

As atomization was treated to the 5^{th} and 10^{th} day, half animals (5) of every group were dissected, then the lung tissues were weighed and the lung coefficients were calculated according to the weight. Pathological changes of appearance and the lung tissue were observed respectively by naked eye and pathological section.

### Experimental results:

(1)After atomization was treated for 5 days, the mice were dissected, the mice lung of model group were found dark red, the color of individual lung lobes were more deeper than others, and the lung of reference group were bright pale pink. There was obvious contrast between model group and reference group and evident change in the appearance of the administrated mice lung. Lung coefficient was shown in Tab.7.

**Tab.7 Lung coefficient after atomization was treated for 5 days**

| Group | Animal count | Weight (g) | Lung coefficient (g/g) ×10⁻³ | P value |
|---|---|---|---|---|
| Reference group | 5 | 30.2±1.6 | 6.4±0.69 | <0.01 (compared with |
| Model group | 5 | 24.5±2.7 | 10.5±1.60 | reference group) >0.05 (compared with |
| High dosage group | 5 | 29.4±3.3 | 9.1±1.00 | model group) |
| Median dosage | 5 | 29.2±2.4 | 7.8±0.65 | <0.01 (compared with |
| group Low dosage group | 5 | 29.6±1.6 | 7.30±0.43 | model group) <0.01 (compared with model group) |

After atomization was treated for 5 days, the weight of the model group was still obviously lower than the weight of reference group, and the weight of the administrated groups were recovered. Lung coefficient of model group obviously increased. The administrated groups except the high dosage group, lung coefficient of median and low dosage group significantly decreased comparing with model group (shown in Tab.7).
The results of pathological section showed that alveolar wall of 4/5 of model group were diffusedly thickened which caused alveolar blocked by pressure and many monocytes and part of leukocyte were infiltrated. 2/5 of high dosage group were changed pathologically, one appeared infiltration of monocytes around bronchiole, the other appeared thickening of alveolar wall at many small focuses of infection accompanied with infiltration of monocytes. The rest 3/5 were normal. 5/5 of median dosage group kept normally. 4/5 of low dosage group kept normally, but one appeared thickening of alveolar wall and infiltration a few monocytes around bronchiole (shown in Fig.3).
Pathohistology observation of bronchiole and bronchia in lung:
Reference group: There was not abnormality in Bronchiole, bronchia, terminal bronchiole, respiratory bronchus and alveolar wall. Model group: There were a large number of destructured phlegmasia cells in bronchiole and bronchia, infiltration of many monocytes and lymphocytes in the corresponding bronchus wall, a little pink mucus accompanied with a few monocytes, pseudostratified epithelium and simple columnar epithelium in part bronchiole and bronchia, and spalling of medium stratum columnar epithelium and cubical epithelium in terminal bronchiole. High dosage group: There were little infiltration of a few monocytes and lymphocytes in bronchus between lobules, and a few phlegmasia cells and secretion in part bronchioles. Median dosage group: There were little infiltration of a few monocytes and lymphocytes around part bronchioles, intactness of upper strata wall of most bronchioles tunica mucosa, spalling of a few epithelium mucosae. Low dosage group: There were two spalling of epithelium mucosae in bronchioles and bronchia of one lung tissue, a little erythrocytes in bronchiole lumina of another lung tissue, and normality in the others. Bronchiole and bronchia in the rest lung were normal.

(2)After atomization was treated for 10 days, the mice were dissected, the mice lung of model group were found dark red and obvious contrast in color compared with reference group. Moreover a animal of model group died on 9^{th} day, there were heavy degree of pathological change in lung, and lung lobe changed to dark black. The appearance of administrated mice groups changed a lot (shown in Fig.4), Lung coefficient of groups was shown in Tab.8.

**Tab.8 Lung coefficient after atomization was treated for 10 days**

| Group | Animal count | Weight (g) | Lung coefficient (g/g)×10⁻³ | P value |
|---|---|---|---|---|
| Reference group | 5 | 31.6±2.4 | 6.38±0.68 | <0.01 (compared with ref- |
| Model group | 4 | 28.0±1.9 | 9.90±0.66 | erence group) |
| High dosage group | 5 | 31.2±2.0 | 8.32±1.84 | >0.05 (compared with model group) |
| Median dosage group | 5 | 30.6±2.2 | 8.30±1.40 | <0.01 (compared with model group) |
| Low dosage group | 5 | 30.7±3.0 | 7.10±0.61 | <0.01 (compared with model group) |

After atomization was treated for 10 days the weights of model group were significantly lower than that of reference group (p<0.05). The weights of administrated group were closed to reference group. Lung coefficient of model group was significantly higher than that of reference group (p<0.01), Lung coefficient of administrated group appeared decreasing tendency. Only low dosage group appeared significance (p<0.01) (shown in Tab.8).

The above experimental results showed that human lysozyme was validated effective on mice model of bronchus lung injury caused by inspiring hydrochloric acid. It was best to treat with atomization at clinical dose of people. There was not obvious relationship between dose and effect.

### E. Determination of the prevention and inhibition of recombinant human lysozyme medicine on coronavirus in vitro.

1, Verification medicine: recombinant human lysozyme, protein level is 4.3845mg, was provided by Changchun Qilong biotechnology institute.
2, Positive reference medicine: Ganciclovir injection, Batch No.020802, was provided by Hubei Keyi medicine Limited Company.
3, Cell: Henrietta Lacks strain of cancer cells (HELA) was offered by this room.
4, Virus: No.04 Coronavirus isolated strain (No.04 serum sample of SARS patient) was provided by You'an hospital.
5, CO₂ incubator was provided by NUAIR US AUTO FLOW.
6, (XSZ-D2) was produced by Chongqing optical instrument factory.
7, Other reagent and apparatus were offered by this room.

### Test methods

1. Determination of the toxicity of recombinant human lysozyme to HELA cell:
   HELA cells were seeded on 96-well culture plates at the concentration of 400,000/ml and grown at 37 in a 5% CO₂ incubator for 2 hours. 100µl recombinant human lysozyme which were double-diluted from 6000 ug/ml to 11.7ug/ml and positive reference medicine which were double-diluted from 6000 ug/ml to 11.7ug/m1 were respectively added to 3 wells per concentration. At the same time the reference cells were cultivated at 37 in a 5 % CO₂ incubator for 6 days. Inverted microscope was used everyday to observe the cytopathic effect (CPE). The cytopathic effect under 25 % was recorded as " + ", the cytopathic effect under 26 % ~ 50 % was recorded as " + + " , the cytopathic effect under 51 % ~ 75 % was recorded as "+++" and the cytopathic effect under 76 % ~ 100 % was recorded as "++++". Drug median toxic dose (TD₅₀) and minimum toxic dose (TD₀) were calculated by using Reed-Muench method.
2. Determination of the toxicity of No. 04 coronavirus in HELA cells:
   The separation of No. 04 Coronavirus (separation of SARS patient's serum): The HELA cells were seeded on cultivation tube at the concentration of 400,000/ml and grown at 37 in 5% CO₂ incubator for 24 hours. After removal of cultivation liquid, 0.2ml SARS patient's serum were added to HELA cells and cultivated at 33°C in rotor for 5 hours, and then supplemented with 1 ml maintenance liquid. At the same time reference cells were cultivated at 33 in rotor for 5 to 7 days. After the appearance of CPE, the method of PCR was used to detect coronavirus. NO.04 sample was positive and then confirmed as separation strain of coronavirus. Using the terminal dilution method to purify the virus twice, the virus was still positive. The results of the determination of two SARS patient's serum by the immunofluorescence were rigidity of IgM and multiplication of lgG4, so the virus was confirmed as coronavirus. The method of CPE was adopted to determine the virus potency.
   CPE method for virus:
      HELA cells were seeded on 96-well cultivation plates at the concentration of 400,000/ml and grown at 37 in a 5% CO₂ incubator for 24 hours. 100µl virus liquid which was diluted to 5 concentrations from 10⁻¹ to 10⁻⁵ were added to three holes per concentration respectively. At the same time reference cells were cultivated at 37 in a 5 % CO₂ incubator for 5 to 7 days. Inverted microscope was used everyday to observe the cytopathic effect (CPE) The cytopathic effect under 25 % was recorded as " + ", the cytopathic effect under 26 % ~ 50 % was recorded as " + + " , the cytopathic effect under 51 % ~ 75 % was recorded as "+++" and the cytopathic effect under 76 % ~ 100 % was recorded as "++++". Drug median tissue culture infective dose (TCID₅₀) were calculated using Reed-Muench method.
3. The inhibition of human lysozyme on No.04 Coronavirus in HELA cells
   HELA cells were seeded on 96-well cultivation plates at the concentration of 400,000/ml and grown to monolayer at 37 in 5% CO₂ incubator for 24 hours. After removal of cultivation liquid, coronavirus liquid were added to HELA cells and absorbed at 37 in 5% CO₂ incubator for 2 hours. Afer removal of coronavirus liquid, the serial concentration of recombinant lysozyme medicine which were double-diluted from minimum toxic dose (TD₀) to 10 concentration (750µg/ml~1.46µg/ml) and positive reference Ganciclovir which were double-diluted from minimum toxic dose (TD₀) to 10 concentration (6000µg/ml~1.46µg/ml) were respectively added to 3 holes of HELA cells per concentration. At the same time reference cells and reference virous were cultivated at 37 in 5 % CO₂ incubator for 5 to 7 days. Inverted microscope was used everyday to observe the cytopathic effect (CPE) of virus. The determination were ended according to the appearance of "+++-++++". Drug median inhibitory concentration (IC₅₀), minimal inhibitory concentration (MIC) and therapeutic index (TI) were calculated to confirm medicine effects using Reed-Muench method. The determination was repeated three times.
4. The prevention of human lysozyme on No.04 Coronavirus in HELA cells:
   HELA cells were seeded on 96-well cultivation plates at the concentration of 400,000/ml and grown to monolayer at 37°C in 5% CO₂ incubator for 24 hours. After removal of cultivation liquid, the serial concentration of recombinant lysozyme medicine which were double-diluted from minimum toxic dose (TD₀) to 10 concentration (750µg/ml~1.46µg/ml) and positive reference Ganciclovir which were double-diluted from minimum toxic dose (TD₀) to 13 concentration (6000µg/ml~1.46µg/ml) were respectively added to 3 holes of HELA cells per concentration and absorbed at 37°C in 5% CO₂ incubator for 2.5 hours. Then 100 TCID₅₀ coronavirus liquid were added to HELA cells. At the same time reference cell and reference virous were cultivated at 37°C in 5 % CO₂ incubator for 5 to 7 days. At the same time normal cell reference and virous reference were cultivated at 37°C in 5 % CO₂ incubator for 5 to 7 days. Inverted microscope was used everyday to observe the cytopathic effect (CPE) of virus. The determination were ended according to the appearance of "+++-++++". Drug median inhibitory concentration (IC₅₀), minimal inhibitory concentration (MIC) and therapeutic index (TI) were calculated to confirm medicine effects using Reed-Muench method. The determination was repeated three times.

### Results of the test

1. The results of the toxicity of Recombinant Human Lysozyme to HELA cells:
   Recombinant Lysozyme: minimum toxic dose (TD₀) was 750±0µg/ml, median toxic dose was 1500±0µg/ml (TD₅₀).
   Positive reference Ganciclovir: minimum toxic dose (TD₀) was 6000±0µg/ml, median toxic dose was 6000±0µg/ml (TD₅₀).
2. The results of the toxicity of No.04 Coronavirus to HELA cells:
   No.04 Coronavirus: median tissue culture infective dose (TCID₅₀) was 10⁻³.
3. The inhibition of Recombinant Human Lysozyme on No.04 Coronavirus to HELA cells (The following data was the mean value of the results of thrice test)
   Recombinant Human Lysozyme: Method of CPE, the median inhibitory concentration (IC₅₀) was 23.4±0µg/ml, the minimum inhibitory concentration (MIC) was 46.8±0µg/ml, and the therapeutic index (TI) was 16.
   Positive reference Ganciclovir: Method of CPE, the median inhibitory concentration (IC₅₀) was 11.7±0µg/ml, the minimum inhibitory concentration (MIC) was 23.44±0µg/ml, the therapeutic index (TI) was 256.
4. The prevention of Recombinant Human Lysozyme on No.04 Coronavirus to HELA cells (The following data is the mean value of the results of thrice test)
   Recombinant Human Lysozyme: Method of CPE, the median inhibitory concentration (IC₅₀) was 5.9±0µg/ml, the minimum inhibitory concentration (MIC) was 11.7±0µg/ml, and the therapeutic index (TI) was 64.
Positive reference Ganciclovir: Method of CPE, the median inhibitory concentration (IC₅₀) was 11.7±0µg/ml, the minimum inhibitory concentration (MIC) was 23.44±0µg/ml, the therapeutic index (TI) was 256.

The above-mentioned results of the test indicated that recombinant human lysozyme had preventive and inhibitory effects. It was confirmed that the preventive effects on Coronavirus was superior to the inhibitory effects on Coronavirus.
Concrete mode of execution:

### Example 1

Prepairation of Recombinant Human Lysozyme: After the high-pressure sterilization of nutritive medium, Recombinant Human Lysozyme strain were inoculated and grown at 250 rounds per minute at 20°C for 36 hours on the thermostatical rotor. The seed tank were cultivatied firstly, then the product tank were cultivatied. The cultivation medium finished expression of fermentation were extracted and purified. The concentrated protein were freeze-dried and then conserved after determination of the activity of protein. Recombinant Human Lysozyme (95%~99% in purity, 30000U/mL/mg in activity) were prepared for use.

### Preparation of Recombinant Human Lysozyme aerosol:

Recombinant Human Lysozyme (95%~99% in purity, 30000U/mL/mg in activity) were freeze-dried into aerosol capsule contained 30000u per granule and then loaded in the pump of aerosol capsule manufactured from Wakawa, France. The process of preparation should be done according with pharmaceutical regulations in the pharmaceutical factory consistent with GMP. This medicine was directively administrated to lung by breathing and used for the infection of the upper or lower respiratory tract caused by drug-resistant virus, Gram-positive bacteria and Gram-negative bacteria, such as the disease of faucitis, tracheitis and pneumonia etc.

### Example 2

Recombinant Human Lysozyme aerosol were prepared using the method mentioned in example 1. Recombinant Human Lysozyme (97% in purity, 30000U/mL/mg in activity) were prepared to aerosol contained 30000u/ml per aerosol and then loaded in atomization machine manufactured from Berry company, Germany. The process of preparation should be done according with pharmaceutical regulations in the pharmaceutical factory consistent with GMP. This medicine was directively administrated to lungs by breathing and used for the infection of the upper or lower respiratory tract caused by drug-resistant virus, Gram-positive bacteria and Gram-negative bacteria, such as the disease of faucitis tracheitis and pneumonia etc.

### Example 3

Recombinant Human Lysozyme aerosol were prepared using the method mentioned in example 1. Recombinant Human Lysozyme (98% in purity, 30000U/ mg in activity) were prepared to aerosol contained 220000u/ml per aerosol and then loaded in the pump of aerosol capsule manufactured from Wakawa, France. The process of preparation should be done according with pharmaceutical regulations in the pharmaceutical factory consistent with GMP.

### Example 4

Recombinant Human Lysozyme aerosol were prepared using the method mentioned in example 1. Recombinant Human Lysozyme (95% in purity, 30000U/ mg in activity) were prepared to aerosol contained 100000u/ml per aerosol. The process of preparation should be done according with pharmaceutical regulations in the pharmaceutical factory consistent with GMP.

## Claims

1. Characteristic of Human lysozyme medicine is that form of medication is aerosol (powder or liquid spray).

2. According to the description in the article 1, characteristic of human lysozyme medicine is that it contains Human lysozyme of 1500-3000000 U/ml in activity.

3. According to the description in the article 1, characteristic of Human lysozyme medicine is that it is consisted of 0.28g span85, 0.28g oleinic acid acetic acid, 10g 134A 80% 10~20mM (pH6.5~7.5) phosphate buffer and 5~25% propylene glycol.

4. According to the description in the article 1, characteristic of Human lysozyme medicine is that Human lysozyme is recombinant human lysozyme expressed by genetic engineering. Genetic engineering expression with amino terminus of human lysozyme connected recombinant human lysozyme modified with (aminoglutaminic acid - 2-aminopropionic acid) 2 or (aminoglutaminic acid - 2-aminopropionic acid) 3, or genetic engineering expression or chemosynthesis mutant recombinant human lysozyme.

5. According to any description in the articles 1-4, the application of human lysozyme medicine to preventing or curing the disease caused by virus, bacteria, drug-resistant bacteria and chemical chlorine gas.

6. According to any description in the articles 1-4, the application of human lysozyme medicine to preventing or curing pneumonia, tracheitis or amygdalitis caused by virus, bacteria, drug-resistant bacteria and chemical chlorine gas.

7. According to the any description in the articles 1-4, the application of human lysozyme medicine to curing tracheitis, pneumonia, lung abscess caused by virus(RNA or DNA virus) or severe acute respiratory syndrome (SAES).

8. According to the any description in the first to forth articles, characteristic of preparation method of Human lysozyme medicine is that freeze-dried powder of recombinant human lysozyme (95%~99% in purity, 30000U/mL/mg in activity) is prepared to aerosol capsule (15000u~300000u/granule) and then loaded in the pump of aerosol (powder spray) capsule. The preparation should be done according with pharmaceutical regulations in the pharmaceutical factory consistent with GMP.

9. According to the any description in the first to forth articles, characteristic of preparation method of human lysozyme medicine is that genetic recombinant human lysozyme (95%~99% in purity, 30000U/mL/mg in activity) were prepared to aerosol (liquid spray)(15000u~300000u/ml/aerosol). The preparation should be done according with pharmaceutical regulations in the pharmaceutical factory consistent with GMP.

## Amended claims

### Amended claims under Art. 19.1 PCT

**1.** Characteristic of Human lysozyme medicine is that form of medication is aerosol or powder spray, it is consisted of Human lysozyme (1500-3000000 U/ml in activity), 0.28g span85, 0.28g oleic and acetic , 10g 134A, 80% 10~20mM (pH6.5~7.5) phosphate buffer and 5~25% propylene glycol.

**2.** According to the description in the article 1, characteristic of Human lysozyme medicine is that it is recombinant human lysozyme expressed by genetic engineering, Genetic engineering expression with amino terminus of human lysozyme connected recombinant human lysozyme modified with (aminoglutaminic acid - 2-aminopropionic acid) ₂ or (aminoglutaminic acid - 2-aminopropionic acid)₃, or genetic engineering expression or chemosynthesis mutant recombinant human lysozyme.

**3.** According to the description in the articles 1-2, the application of human lysozyme medicine to preventing or curing the disease caused by virus, bacteria, drug-resistant bacteria and chemical chlorine gas.

**4.** According to the description in the articles 1-2, the application of human lysozyme medicine to preventing or curing pneumonia, tracheitis or amygdalitis caused by virus, bacteria, drug-resistant bacteria and chemical chlorine gas.

**5.** According to the description in the articles 1-2, the application of human lysozyme medicine to curing tracheitis, pneumonia, lung abscess caused by virus(RNA or DNA virus) or severe acute respiratory syndrome (SAES).

**6.** According to the description in the articles 1-2, characteristic of preparation method of Human lysozyme medicine is that freeze-dried powder of recombinant human lysozyme (95%~99% in purity, 30000U/mL/mg in activity) is prepared to powder spray capsule (15000u~300000u/granule) and then loaded in the pump of powder spray capsule. The preparation should be done according with pharmaceutical regulations in the pharmaceutical factory consistent with GMP.

**7.** According to the description in the articles 1-2, characteristic of preparation method of human lysozyme medicine is that genetic recombinant human lysozyme (95%~99% in purity, 30000U/mL/mg in activity) were prepared to aerosol (15000u~300000u/ml/aerosol). The preparation should be done according with pharmaceutical regulations in the pharmaceutical factory consistent with GMP.
